# EUROPEAN PATENT SPECIFICATION

(11) **EP 1 694 404 B1**
(45) Date of publication and mention of the grant of the patent: **09.02.2011**
(21) Application number: 04797709.5
(22) Date of filing: 08.11.2004
(51) Int. Cl.: A61N 1/36

(54) **ELECTROTHERAPY APPARATUS**
ELEKTROTHERAPEUTISCHES GERÄT
APPAREIL D'ELECTROTHERAPIE

(30) Priority: 07.11.2003 EP 03025663
(43) Date of publication of application: 30.08.2006
(73) Proprietor: CardioLa Ltd., 8404 Wintherthur (CH)
(72) Inventor: LAPANASHVILI, Larry, CH-8404 Winterthur (CH)
(74) Representative: Morgan, James Garnet
(86) International application number: PCT/EP2004/012617
(87) International publication number: WO 2005/044372

(56) References cited:
- EP-A- 0 847 776
- EP-A- 1 136 097
- WO-A-01/13990
- US-A- 4 541 417
- US-A- 4 688 574

## Description

The present invention relates to Electrotherapy apparatus for applying electrical stimulation to a muscle or group of muscles of a human or other mammal, wherein said electrical stimulation comprises trains of electrical pulses, said electrical stimulation having parameters comprising a pulse repetition frequency, a duration of each pulse or group of pulses and a time offset of the start of the train of pulses relative to a predicted end of a T-wave of an electrocardiogram derived from said human or mammal, said offset lying in a range from 5 % of the R-R path length of the preceding heart cycle, or of an average value of the R-R path lengths of a plurality of preceding heart cycles, or of a representative R-R path length, before the predicted end of said T-wave up to 45 % of the R-R path length of the preceding heart cycle, or of an average value of the R-R path lengths of a plurality of preceding heart cycles, or of a representative R-R path length, after the predicted end of the T-wave.

Electrotherapy apparatus of the initially named kind is described in the international patent application with the publication number WO 01/13990 A1. In this reference provision is made for varying the parameters of electrical stimulation.

It has been found that this type of electrotherapy leads to extremely beneficial effects with respect to the heart of the person or mammal and, depending on precisely how the electrotherapy is carried out, can also be used for curing a whole spectrum of adverse conditions.

The object of the present invention is to improve the performance of the previously described apparatus to provide a significant improvement in hemodynamics, i.e. the blood circulation in the body and through the heart, and in particular to prolong the length of time a treatment can be carried out without the muscles contracted by the electrostimulation loosing their responsiveness to the applied electrical stimulation by becoming accustomed to it.

In order to satisfy this object there is provided an apparatus in accordance with claim 1.

It has namely been found that by regularly changing or randomly varying the pattern of electrical stimulation applied to an electrode the muscle associated with it does not become acclimatised to the stimulation and unresponsive to it. This phenomenon is similar to the way people become accustomed to regularly repeating noises in the sense that after a while they no longer react to it or hear it. By preventing the muscles affected by electrostimulation from becoming accustomed to it their response in the sense of repeated muscle contractions can be prolonged indefinitely. Thus users with portable electrotherapy apparatus could use it for years on end without the apparatus loosing its effectiveness as a result of the patients muscles becoming unresponsive.

The concept of the invention is specifically intended for counterpulsation electrotherapy where long treatment times can be particularly beneficial for the therapeutic treatment of the heart.

The apparatus preferably has a plurality of output channels for applying electrical stimulations to a plurality of active electrodes provided on the person being treated.

The reason for this is as follows:

It has been found that if a muscle is subjected to contraction signals once every heart cycle, then it can become fatigued. On the other hand, the present invention is not critical with respect to the muscle to which the contraction is applied. Accordingly, it is preferable to provide a plurality of active electrodes, for example four active electrodes, which each affect a separate muscle of a group of muscles or a region of muscles on the human body. Each channel of the electrotherapy apparatus is connected to a respective one of said active electrodes. Thus, if four active electrodes are present, the first channel can be connected to the first electrode and can provide electrical stimulation for a first muscle, the second channel can be connected to a second electrode and provide electrical stimulation for a second muscle, the third channel can be connected to a third electrode and provide electrical stimulation for a third muscle, and the fourth channel can be connected to a fourth electrode and provide electrical stimulation for a fourth muscle. This means that each muscle is stimulated only once every four heart cycles and there is therefore a rest period of three heart cycles between each electrical stimulation of any particular muscle. This avoids fatigue of the muscles.

The electrotherapy apparatus thus applies the same electrical stimulation to each output channel in turn, each electrical stimulation comprising the initial stimulating pulses and the further stimulating pulses. Channel 1 is activated after one complete heart cycle has been detected, channel 2 is activated once a subsequent heart cycle has been detected and so on. The timing of the electrical stimulation signals applied to each channel is based on the R-R path length of the preceding heart cycle or on an average R-R path length of a plurality of preceding heart cycles. The reference in the above paragraph to the "same electrical stimulation does not mean that the electrical stimulation applied is invariable but rather that the pattern of stimulation that is used is generally, but not necessarily used for a period of time before it is replaced with a different pattern.

This technique as described above also makes it possible to use different electro-stimulation signals, i.e. different stimulation signal shapes and values in each channel, which can also be beneficial under some circumstances.

An electrotherapy apparatus is particularly preferred in which a plurality of channel groups is provided, with each channel group comprising a plurality of channels. Each channel group preferably has the same number of channels. For example two or three channel groups can be provided and each channel group can comprise four channels.

There are a variety of special ways in which such an apparatus can be operated as will be described later.

Further advantageous embodiments of the electrotherapy apparatus can be seen from the further claims.

The invention will now be described in more detail by way of example only with reference to the accompanying drawings in which Figs. 1 to 4 are generally similar to Figs. 1 to 4 of the above-mentioned document WO 01/13990, but with certain modifications in Figs. 2a and 4. More specifically there is showing in the Figures:

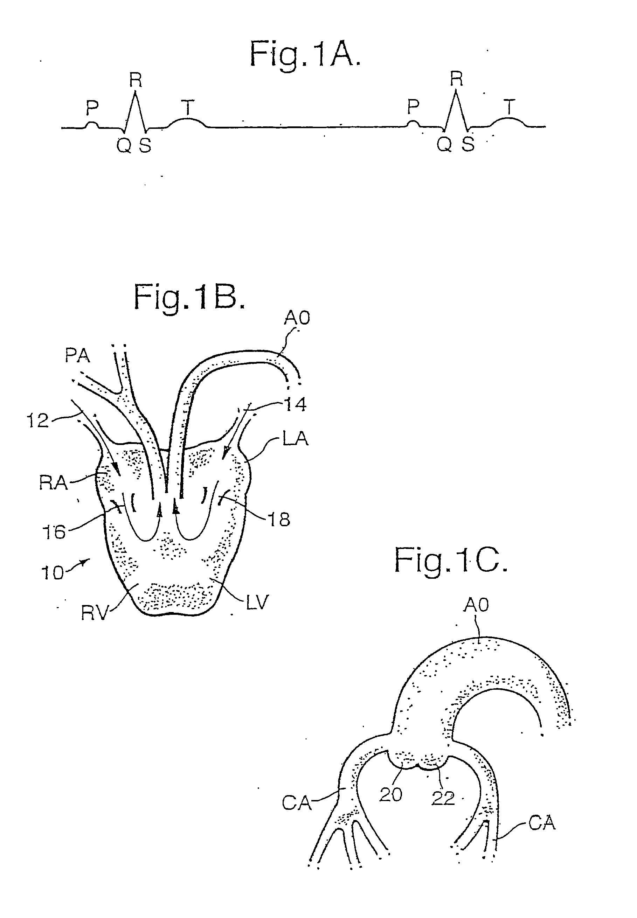

- Fig. 1A: a schematic diagram illustrating a typical electrocardiogram,
- Fig. 1B: a schematic diagram of the human heart,
- Fig. 1C: an enlarged view of the aorta at the junction with the heart and with the coronary arteries,

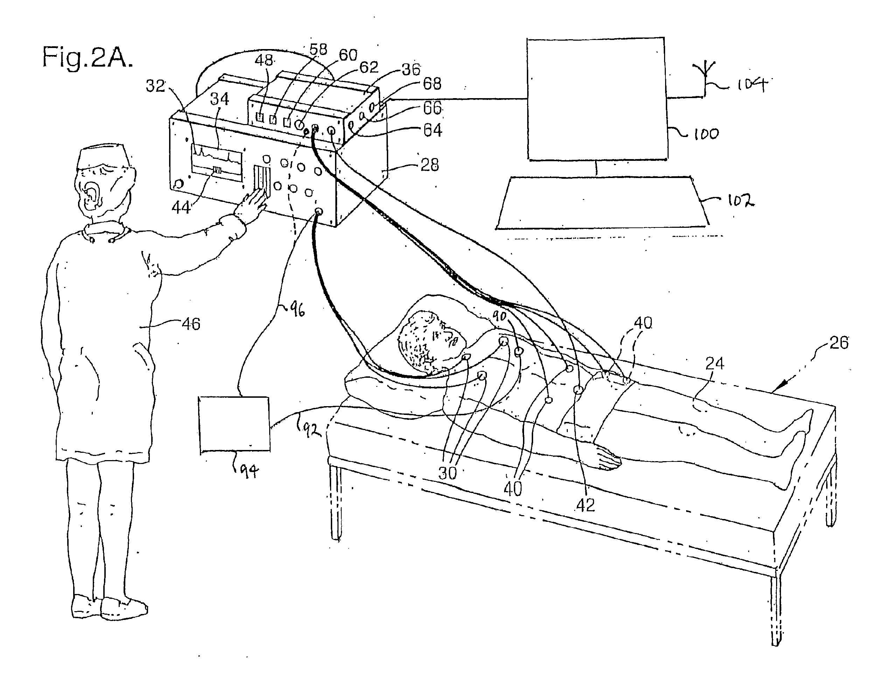

- Fig. 2A: a schematic diagram of a first variant of an apparatus for applying electrostimulation,

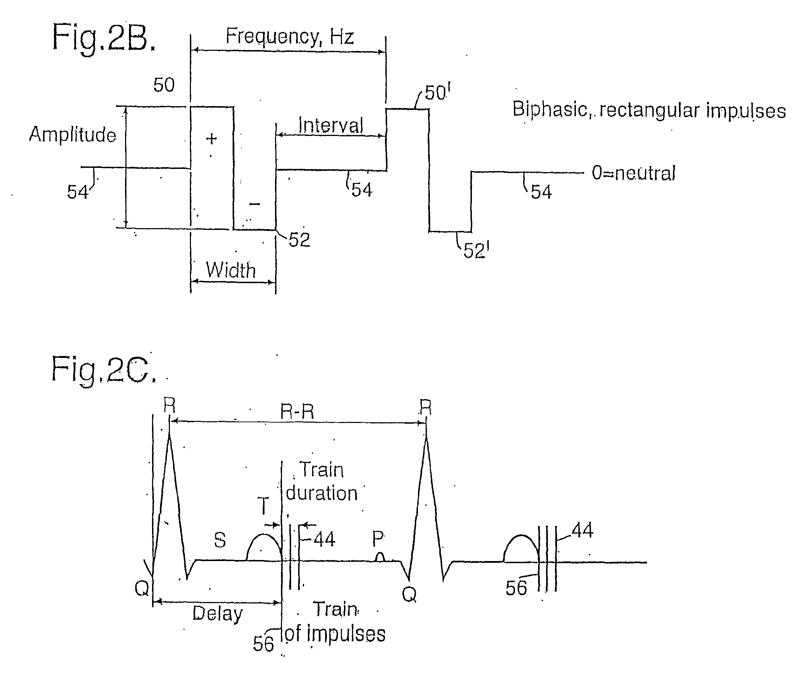

- Fig. 2B: a graph illustrating the terminology used to describe a biphasic rectangular impulse,
- Fig. 2C: a graph illustrating the timing of the pulses applied to a patient in the counterpulsation mode to achieve cardioresonance,

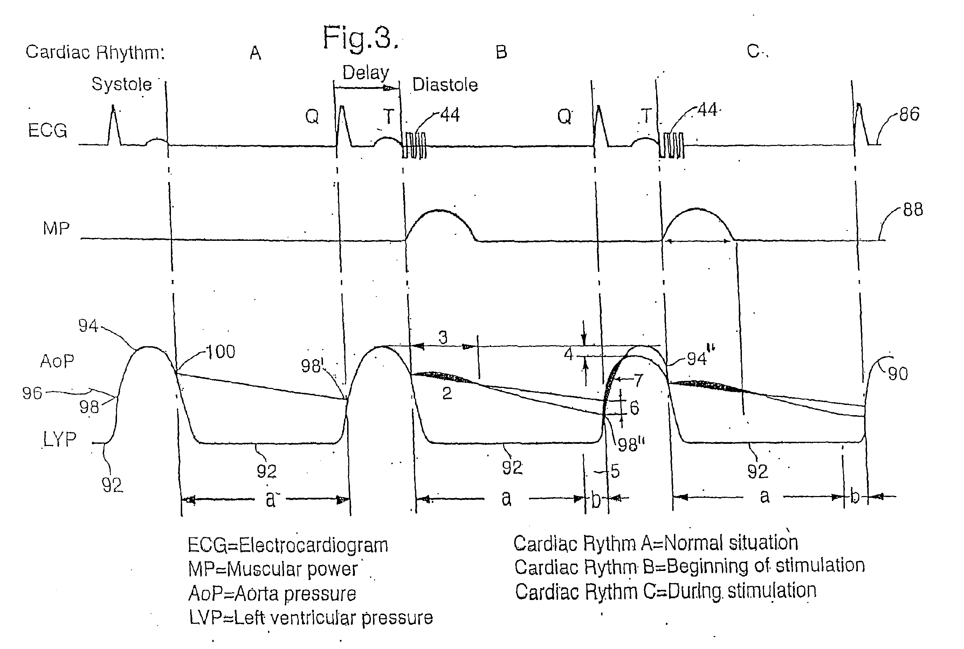

- Fig. 3: a set of diagrams showing the effect of the apparatus of the invention on the operation of the heart of a patient,

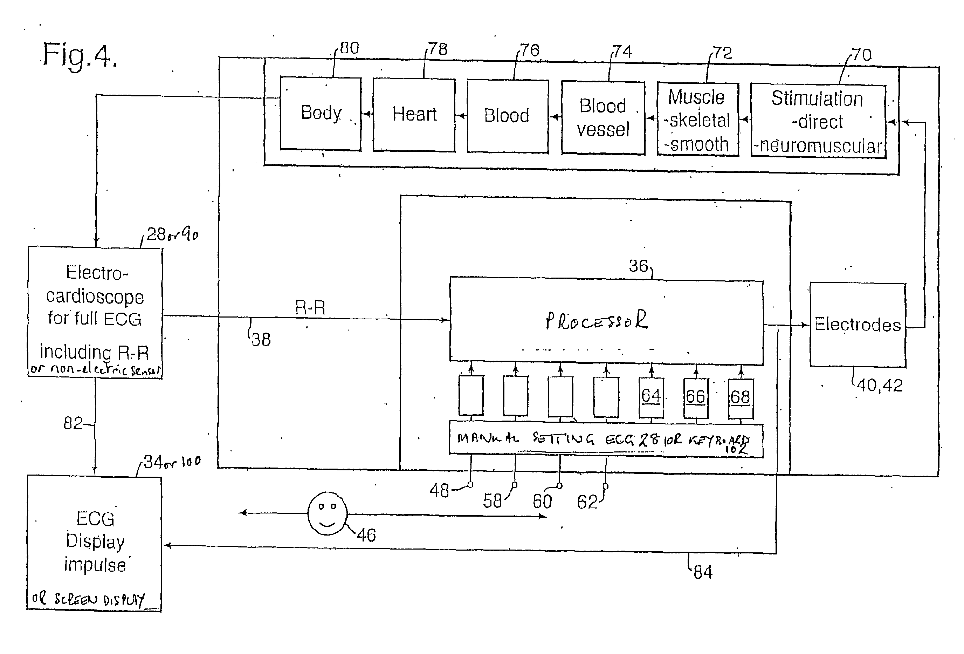

- Fig. 4: a schematic diagram illustrating the operation of an apparatus similar to that of Fig. 2A,

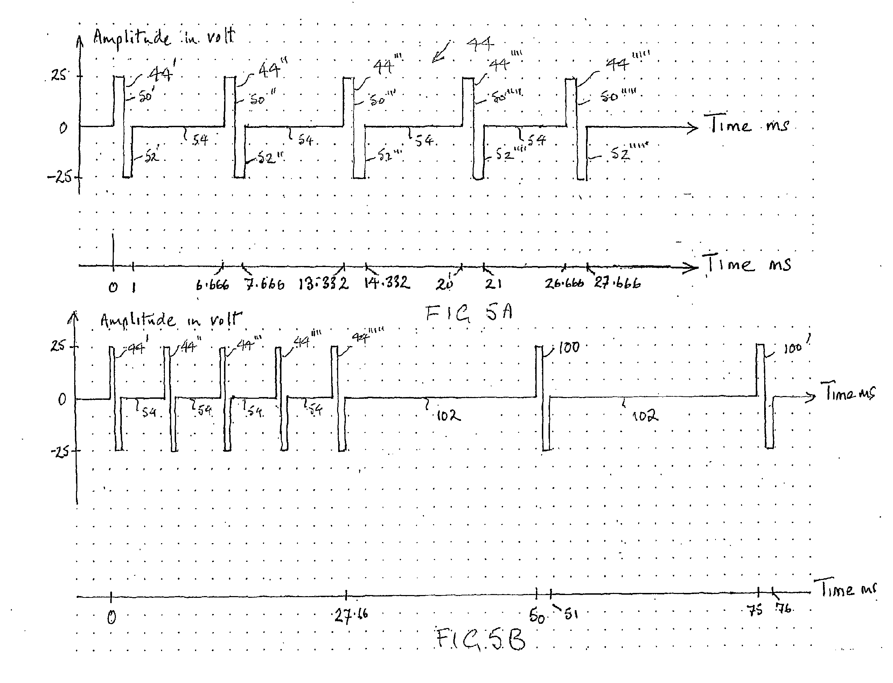

- Fig. 5A: a diagram to explain the preferred train of initial electrical stimulation pulses generated by the processor,
- Fig 5B: a diagram to explain the relationship between the train of initial electrical stimulation pulses and the further electrical stimulation pulses generated by the processor,

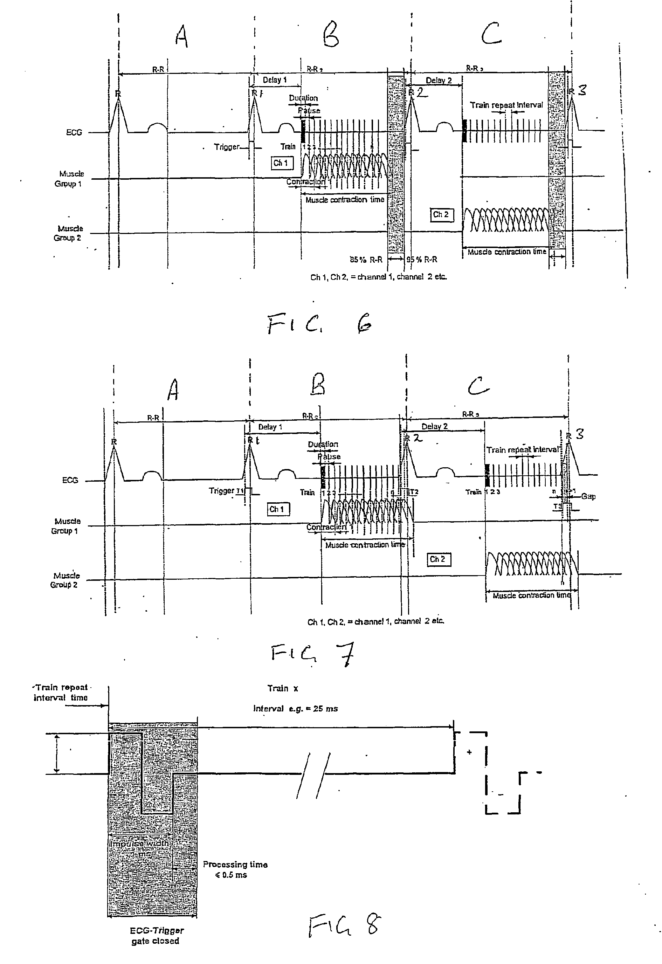

- Fig. 6: a diagram explaining how the electrical stimulation pulses are timed in an attempt to ensure that the muscle ontraction is concluded before the next R-peak,
- Fig. 7: a further diagram explaining what happens when the next R-peak arrives earlier than expected,
- Fig. 8: a diagram illustrating the concept of interference windows,

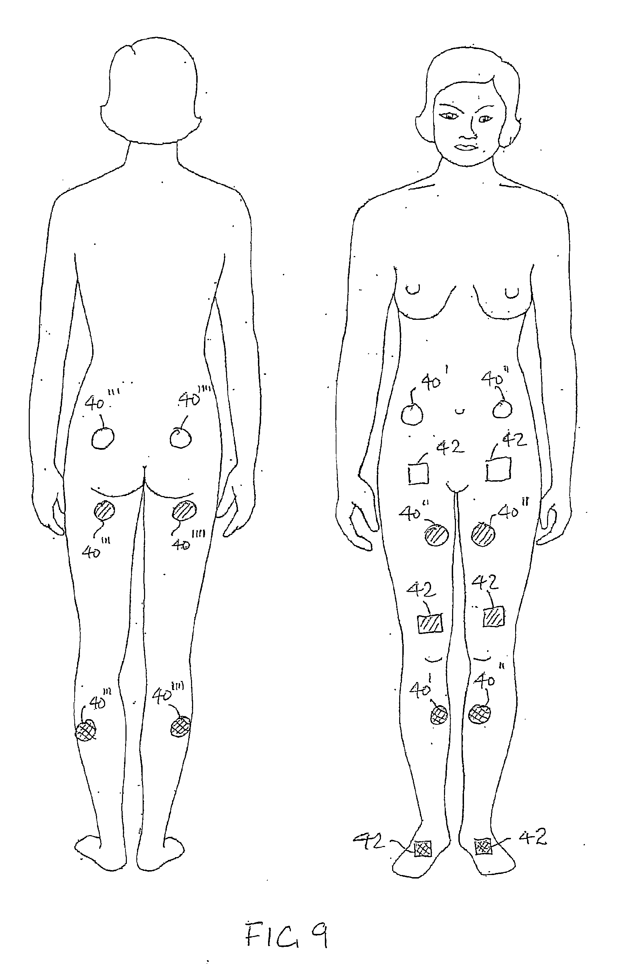

- Fig. 9: a diagram illustrating one possible placement of active electrodes on a person and

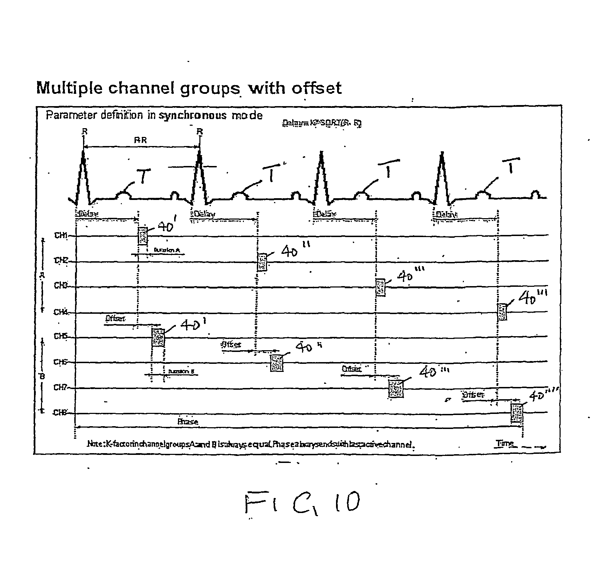

- Fig. 10 a: diagram to explain how the electrodes of Fig. 9 can be supplied with stimulation signals.

In the following the background to the present invention will first be described with reference to Figs. 1 to 10 and then the present invention will be explained in more detail with reference to Fig. 2A.

Turning now to Figs. 1A, 1B and 1C, a brief description of the normal operation of the human heart will be given in order to facilitate an understanding of the present invention.

The heart 10 shown in Fig. 1B has four chambers, namely the right atrium RA, the right ventricle RV, the left ventricle LV, and the left atrium LA. Venous blood returning to the heart flows into the right atrium, then into the right ventricle and passes to the lungs via the pulmonary artery PA. In the lungs the blood picks up oxygen and returns to the left atrium LA, as indicated by the arrow 14. From there, the oxygenated blood passes into the left ventricle, and then into the aorta AO where it starts on its journey through the so-called big circulation around the body. The circulation from the right ventricle to the lungs and then to the left atrium is called the minor circulation.

The operation of the heart is associated with electrical signals, which are shown on the electrocardiogram of Fig. 1A. The point P signifies the contraction of the two atriums RA and LA, which pushes blood into the respective ventricles RV and LV via the respective valves 16 and 18, which act as non-return valves. The section of the electrocardiogram starting with Q and ending with T is referred to as the systole and represents the ventricle contraction which serves to expel blood from the right ventricle into the pulmonary artery, and from the left ventricle into the aorta. During this contraction, the valves 16 and 18 are closed to prevent reverse flow into the right atrium and left atrium. The section TQ is referred to as the diastole, meaning the relaxation or expansion of the ventricles. The heart is supplied with oxygenated blood via the coronary arteries CA, which branch off from the aorta just upstream of the valves 20, 22, which close to prevent blood returning from the aorta to the left ventricle during the diastolic phase. Clearly the heart, itself a muscle, must be supplied with oxygenated blood to keep the muscles working. The heart is supplied with this oxygenated blood via the coronary arteries CA during diastole. At T the valves 20, 22 of the aorta AO are closed and at this time the blood pressure in the aorta causes blood to enter the coronary arteries CA. Accordingly, an increase of the pressure in the aorta AO during diastole favors the coronary arteries.

As will be seen from the following, one of the important results associated with the present invention is a small increase in pressure in the aorta during diastole and this has been found to have a profound effect on the operation of the heart muscle.

Fig. 2A shows an illustration of a basic apparatus which has been used for the testing of the present invention.

As shown in Fig. 2A, a patient 24 is shown lying on a bed 26 and is connected to an electrocardioscope 28 via (in this embodiment) three sensing electrodes 30, which enable the electrocardioscope to show the ECG trace 32 for the particular patient 24 on the display 34. From the information available to the electrocardioscope through the three electrodes 30, a signal is extracted corresponding to the repetition frequency of the path R-R of the ECG trace of Fig. 1A. That is to say, this signal represents the frequency at which the patient's heart beats, i.e. his pulse rate.

This signal is fed via a line 38, which is not shown in Fig. 2A but which is schematically illustrated in the diagram of Fig. 4 relating to the operation of the apparatus of Fig. 2A, to a processor 36 with an associated trigger system. In this embodiment the trigger system is embodied in the processor and suitable software is provided so that the trigger system delivers an initial electrical stimulation in the form of a train of biphasic rectangular pulses to the patient 24 via the active electrodes 40, of which four are shown in Fig. 2A. The precise shape of the train of biphasic rectangular pulses will be described later with reference to Fig. 2B. Although the trigger system is embodied in the processor in this example it could also be a separate unit (not shown) and simply receive trigger signals from the processor. In that case the output channels described here will not generally be present at the processor but at the output side of the separate unit.

The further electrode 42 is a neutral electrode necessary to complete the circuit. As illustrated in Fig. 2C the train of pulses 44 is triggered once per cycle of a patient's heart and is timed to coincide with the end of the T-wave of the electrocardiogram. The train of pulses 44 providing the initial electrical stimulation applied to the patient is also shown on the display 34 of the electrocardioscope, which enables the operator 46 to see the phase relationship between this train of pulses 44 and the electrocardiogram 32.

From the joint display on the screen 34 of the electrocardioscope of the ECG and the train of pulses 44 providing the initial electrical stimulation applied to the patient, the operator 46 can see whether the train of pulses has the appropriate delay relative to the Q-wave to secure the cardioresonance desired.

As noted earlier, the train of pulses is preferably set to start at the expected end of the T-wave. Depending on the circumstances it could, however, be set to start within a window extending from 5 % of the R-R path length of the preceding heart cycle, or of an average preceding R-R path length, before the end of the expected end of the T-wave up to 45 % of the R-R path length of the preceding heart cycle, or of an average preceding R-R path length, after the expected end of the T-wave. The operator 46 is able to adjust the phase for the start of each train of pulses, i.e. the delay, so that it coincides with, e.g., the end of the T-wave. This is one manual input into the processor indicated at 48 in Figs. 2A and 4.

Before discussing the effect the train of pulses 44 applied to the patient has, it is appropriate to discuss the terminology used in this specification with respect to the pulses generated by the input system comprising the pulse generator 36 and the electrodes 40, 42.

The basic output of the processor 36 is shown in Fig. 2B. It can be seen that the train of pulses providing the initial electrical stimulation comprises a plurality of so-called biphasic, rectangular impulses. Each biphasic rectangular impulse has a rectangular positive half pulse 50, and a rectangular negative half pulse 52 immediately following the positive half pulse, so that the impulse width is determined by the width of 50 plus the width of 52. The biphasic impulse 50, 52 of Fig. 2B is then followed by an interval and thereafter by a second biphasic impulse indicated as 50', 52' in Fig. 2B. The distance between sequential positive half waves 50, 50' of the biphasic pulses determines the pulse repetition frequency of the signal. During the interval between sequential biphasic pulses the voltage applied to the electrodes 40 is zero, i.e. is the same as the voltage at the neutral electrode 42, so that no stimulation of the patient occurs. This zero voltage is indicated by 54 in the diagram of Fig. 2B. It will be noted that instead of applying voltages to the electrodes, currents can be applied to them in which case the references above to voltages should be regarded as references to currents.

For the purpose of giving a reasonable example, the amplitude of the output signal of the pulse generator 36, i.e. as applied to the electrodes 40, can vary from a positive amplitude 50 of plus 40 V to a negative amplitude 52 of minus 40 V.

It must be stressed that these values are simply given by way of example and that substantial variations may be made, depending on a whole variety of factors.

So far as the amplitude of the biphasic signal is concerned, it has been found that different patients have different threshold voltages at which they perceive the treatment as being uncomfortable. Thus, one possibility is for the operator 46 to vary the amplitude of the biphasic pulses until the patient perceives them as being slightly uncomfortable and then to reduce the amplitude slightly so that the patient suffers no discomfort.

Generally speaking, an amplitude with a lower limit starting from slightly above zero volts (say two or three volts) is possible. The upper limit depends on whether the patient feels comfortable with the voltage level applied and the resulting current (very high voltages could be used in theory at least, providing the current is restricted to non-damaging values).

The relationship between the pulse width and the pulse interval of each train of pulses determines the total energy input into the muscles stimulated via the electrodes 40, 42. While a ratio of about 1:5 has been found effective for the initial train of pulses 44 providing the initial electrical stimulation, this ratio can be varied substantially and indeed an interval is not absolutely essential. Generally speaking, with all patients a threshold is reached, depending on the pulse amplitude and the ratio of the pulse width to the interval, at which involuntary contractions of the muscle are apparent to a trained observer and the apparatus will usually be operated with amplitudes and ratios of the pulse width to pulse interval at levels at which apparent involuntary muscular contractions do occur, i.e. above the threshold value.

A particularly important reason for using biphasic pulses is to avoid the onset of electrolysis in the tissue affected by the applied impulses. Any effects of this kind which may be triggered during one half pulse are immediately reversed in the next half pulse. Although biphasic rectangular pulses of the kind described above have been found to be satisfactory and currently represent the preferred type of pulses, they are by no means the only possibility. Generally speaking, it is anticipated that the pulses delivered by the pulse generator will be biphasic in the sense that they have some positive going signal component and some negative going signal component. However, it is not out of the question that single phase rectangular pulses can also be used to advantage in some circumstances. It is certainly not essential that the negative half wave is of the same size and shape as the positive half wave. The positive half wave could be of different amplitude and width from the amplitude and width of the negative half wave. Moreover, it is not essential for the pulses to be rectangular pulses. They could be sinusoidal or they could have some other shape if desired.

As is apparent from Fig. 4, the apparatus shown there provides the operator 46 with various different parameters which he can set during the treatment of a patient. The first of these is the delay or impulse delay, which, as shown in Fig. 2C, is the time difference between the Q wave end of a QRS heart signal and the effective start of the impulses, i.e. the start of the train or burst of impulses which commences at the end of the T-wave. The operator 46 has the possibility of adjusting this delay at 48, for example, by varying a potentiometer which determines the delay as a percentage of the measured R-R path length, or by keying in a corresponding input to the processor, which is then put into effect by the programming of the processor. This is an extremely important adjustment in the apparatus of Fig. 2A and 4 for the following reason:

As will be explained shortly, the effect of the pulses is to unload the heart. This manifests itself by a reduction of the pulse rate, i.e. of the frequency of the heart beat. This means that the time between successive R peaks of the ECG trace increases. Not only does R-R increase, but the distance from Q to the end of the T wave also increases because it stands in a known relationship to the time interval R-R. Thus, if the delay were a fixed value, the start of the train of pulses 44 would not always coincide with the end of the T-wave due to the change in the pulse rate. Accordingly, when the operator sets the delay, this does not mean that he sets a specific value for the delay in milliseconds but rather that he specifies the delay as a specific percentage of the measured R-R path length.

The best results are frequently obtained when the delay is timed so that the first train of pulses is initiated at the end of the T-wave. However, beneficial results can also be obtained if the train of pulses starts slightly earlier or later than the end of the T-wave and indeed, in some applications of the apparatus this is a desirable feature, as will become apparent from the later description.

Practically speaking, it is considered desirable to keep the start of the train of electrical stimulating pulses within a window extending from 5 % of the length of the preceding R-R path before the end of the T-wave of an electrocardiogram up to 45 % of the length of the preceding R-R path after the end of the T-wave. Instead of using the preceding R-R path, an average value of the R-R path over a plurality of preceding heart cycles can be used.

Another parameter which can be varied by the operator 46 is the duration of the train of pulses applied to the patient after the end of each T-wave. As shown in Fig. 2C, the duration of a train is defined as the time between the start and the end of the impulses within a train or burst of impulses. This possibility of variation is indicated in Fig. 4 by the reference numeral 58.

The complete train is the package of electric impulses which are repeated one after the other for the time defined by the duration of the train. The number of electric impulses in each train can be varied by varying the output frequency of the biphasic pulses, i.e. the pulse repetition frequency of the biphasic pulses in each train of pulses, i.e. the number of pulses that are repeated per second if the train of pulses were to be one second long. Furthermore, the duration of the train determines how long the stimulation with a given frequency is repeated, i.e. how many impulses are effectively delivered within one heart cycle. This pulse repetition frequency and in particular how it changes during each heart cycle can be varied by the operator 46 at the input 60 in the example of Fig. 2A and Fig. 4. This will be explained later with reference to Figs. 5A and 5B. The other variable which can be readily changed by the operator 46 in the embodiment of Figs. 2A and 4 is the amplitude of the biphasic rectangular impulses, i.e. the maximum difference between the peak value of the positive half cycle 50 and the peak value of the negative half cycle 52, as shown in Fig. 2B. This possibility of adjustment is indicated at 62 in Fig. 4. The amplitude is normally measured as a potential difference in volts. In an alternative embodiment (not shown) it is possible to plot a current curve rather than a voltage curve and to vary the amplitude with reference to the corresponding peak amplitude of the current curve.

In the apparatus of Figs. 2A and 4 there are three further parameters of the pulses which can be varied by the operator 46. The first of these parameters is the pulse width, i.e. the time before the start and end of an electric impulse, as shown in Fig. 2B. The pulse width is selected in the example of Figs. 2A and 4, so that the interval at a pulse repetition frequency of 150 Hz is 5.66 times as long as the pulse width. That is to say by fixing the pulse width the interval will automatically vary as the pulse repetition frequency is varied. If the pulse width is made variable, as it is in some other embodiments, then varying the pulse width automatically results in the interval shown in Fig. 2B varying, on the assumption that the repetition frequency of the pulses of the train of pulses does not change. Box 64 in Fig. 4 relates to the input at which the desired value of the pulse width is selected.

The further boxes 66, 68 in Fig. 4 represent two further parameters of the output of the pulse generator, which in the apparatus of Fig. 2A and Fig. 4 can be varied by the operator 46. Box 66 relates to the impulse form, i.e. the geometric form of the electric impulse resulting when the amplitude of the electric impulse is displayed over the entire impulse width. In the present example this is a biphasic rectangular pulse but it could have different shapes, for example sinusoidal or saw-toothed.

Box 68 refers to the possibility of changing the impulse mode which relates to the alternating mode of how impulse forms are repeated between electric positive and electric negative phase of impulses. In the present example the impulse mode is clearly biphasic, with positive and negative, but otherwise identical electric impulses alternating one after the other. This mode switch would, however, allow the operator to select some other mode, for example two positive half pulses followed by one negative half pulse.

One other aspect should also be mentioned with reference to Fig. 2A. This is the possibility of using a plurality of electrodes 40, 42. As mentioned above, the electrode 42 is a neutral electrode and it is only necessary to provide one such neutral electrode. However, more than one neutral electrode can be used when different areas of the body are treated, in order to allow a neutral electrode to be in the vicinity of each active electrode or each group of active electrodes. For long term treatment of a patient, it is recommended to provide a plurality of active electrodes 40.

The reason is that the human body can become accustomed to the applied pulses and if only one active electrode 40 is provided, i.e. only one electrode to which the biphasic rectangular impulse signal of Fig. 2B is applied, the muscles that are stimulated by the potential between this electrode and the neutral electrode 42 gradually become tired and are stimulated less effectively. By applying the stimulating impulses to the different active electrodes 40 in sequence, it is possible to ensure that the muscles of the muscle group affected by the applied impulses do not become tired. The minimum number of active electrodes for sequencing is two.

Experiments have shown that by applying the output signal of a pulse generator to several electrodes 40 in sequence the treatment can be carried out over a period of many days without problem, and indeed only two electrodes are sufficient for this. However, four electrodes are preferred.

In the experiments done to date the first train of pulses 44 has been applied to the first electrode 40 during one heart cycle, the next train of pulses 44 has been applied to the second electrode during a next heart cycle, the next train to the third electrode during a subsequent heart cycle, the next train to the fourth electrode during a further subsequent heart cycle and the next train to the first electrode during a later heart cycle and so on. However, a sequence of this kind is not essential. It could be perfectly feasible to feed several trains of pulses to one electrode and then to change to the next electrode etc. Random energization of the electrodes with successive pulse trains or groups of pulse trains would also be entirely feasible.

It should be emphasized that there is nothing critical in the placement of the individual electrodes 40 and 42 so far as heart therapy is concerned. Although these are shown in the stomach region of the patient under treatment here, they could be virtually anywhere on the patient's body. It is a surprising aspect that the stimulation of any part of the peripheral vascular system with even small amounts of excitation energy have been found to produce the beneficial effect of the invention. However, if specific areas of the human body are to be treated by electrotherapy, then the electrodes should be placed so that the therapeutic results desired can be achieved in the area of the electrodes. For example, if the buttocks are to be slimmed then it can be beneficial to provide the electrodes in the vicinity of the buttocks.

A more detailed discussion of the types of electrostimulation possible will be given later in the description.

It will be noted that Fig. 4 also shows with a series of boxes how the stimulation input to the patient from the pulse generator affects the body. Box 70 indicates that the stimulation can be direct stimulation or neuromuscular stimulation which is more usual.

Box 72 shows that the stimulation can be applied either to skeletal muscles or to smooth muscles. The effect of applying the stimulation to skeletal or smooth muscles is in both cases to produce a pressure pulsation in a local blood vessel of the peripheral vascular system indicated by the box 74. This local pressure fluctuation propagates via the blood, essentially an incompressible liquid indicated by box 76, to the heart indicated by box 78. Provided the pulses are timed correctly and applied in accordance with the teaching of the present invention, then they have been found to have a significant effect in reducing the heart load, which itself has an effect on the body of the patient indicated by box 80. This effect is picked up by the electrodes 30 of the electrocardioscope.

As noted earlier, a signal corresponding to the pulse rate, for example the R-R signal, is then passed on to the pulse generator and triggers the generation of the biphasic rectangular pulses of the individual pulse trains. The ECG wave form 82 is shown on the display 34 of the electrocardioscope as is the output signal of the pulse generator, as shown by the lines 82 and 84 in Fig. 4. The operator 46 has the ability to vary the impulse delay to ensure that each train of pulses starts at the end of the T-wave of the electrocardiogram or at the position deemed optimal in a particular case.

Fig. 3 gives a graphic representation of the effect of the treatment with the apparatus of the invention so far as the heart is concerned. The topmost curve 86 shows several peaks of an ECG wave form and is divided basically into three sections A, B and C. Section A shows a patient's cardiac rhythm in a normal situation, i.e. without stimulation. Section B shows the cardiac rhythm for the same patient at the start of stimulation and section C shows the cardiac rhythm during continued stimulation. This division into sections A, B, C also applies to the further curves 88 and 90. In curve 86 section B shows the first train of impulses 44 providing the initial electrical stimulation which starts after the end of the T-wave and lasts for about 15 % of the T-Q path. This same wave form repeats in phase C and continues repeating until the stimulation is terminated. The effect of this stimulation is to produce a significant reduction in the patient's heart rate so that the length between successive R positions of the ECG lengthens in the course of time. It will be noted that the R-R pattern in section C is longer than in section A, by a length labeled "b" as shown in curve 90 in Fig. 3.

Curve 88 shows the modulation of the muscular power resulting from the trains of electrical impulses such as 44 providing the initial electrical stimulation. In phase A of line 88, there is no stimulation and accordingly the line is a straight line. The first stimulation occurs in the section B and results in a stimulation of a muscle which affects the peripheral vascular system. It will be noted that the muscle contraction 3 starts at the start of the train of pulses 44 and tends to reach its maximum contraction at the end of the train of pulses and then relaxes over a time period rather longer than the train duration. It will be noted that the initial train of pulses 44 contains a plurality of stimulating electrical impulses but results in a simple muscular contraction. This muscular contraction 3 produces a pressure pulsation in the patient's peripheral vascular system which propagates back to the patient's heart.

The result of this can be seen from the curve 90, which is in fact a composite curve showing the pressure in the aorta and the left ventricular pressure. The left ventricular pressure starts from a base line value 92 and increase s smoothly into a rounded peak 94, which has a value above the base line value 92 from the start of the Q wave until just after the end of the T-wave. Superimposed on this curve is a curve 96 for the pressure in the aorta.

At the point 98 the valves 20, 22 in Fig. 1C open and the pressure in the left ventricle is communicated directly into the aorta so that the pressure in the aorta rises at the same rate and with the same value as the pressure in the left ventricle until the end of the T-wave is reached, i.e. until the point 100 in Fig. 3, where the valves 20, 22 close again and the pressure in the aorta gradually sinks as the blood in it moves through the arteries of the human body. At point 98' the valves 20, 22 open again and the cycle repeats.

The effect of the muscular contraction, indicated by 3 in the curve 88, is to modulate the pressure in the aorta by a pressure wave traveling back to the aorta, from the peripheral blood vessel pulsation induced by the muscle contraction, so that in phase B it is slightly higher - shown as a visible hump - in the region labeled 2 than the corresponding value in phase A of curve 96. However, after the end of the muscular contraction, the pressure in the aorta sinks to lower values than were present in the corresponding section of the pressure curve in phase A.

At the same time it will be noted that the peak 94" of the left ventricular pressure has also reduced relative to the peak value 94 in phase A. The reduction is labeled 4 in Fig. 3.

What this means in practice is that the hump 2 in the pressure in the aorta in diastole results in increased coronary circulation, i.e. more blood and more oxygen is being supplied to the heart muscles, resulting in more energy being made available to the heart. This causes the pulse rate to reduce so that the duration of each heart beat increases from the value a before stimulation by the amount b to the value a + b after prolonged stimulation. The typical measured reduction with various probates is about 10 pulses per minute in the rest mode, for example 70 down to 60, or up to 30 or more at a high pulse rate, for example from 140 to 110, because of an increase of the DPTI/TTI ratio (diastolic blood pressure time index/time tension index).

In addition, the reduction indicated by 4 from the peak value 94 in phase A to the peak value 94" in the phase C represents a fall in the systolic pressure in the left ventricle and thus reducing left ventricular wall tension.

Bearing in mind that the heart load is proportional to the pulse rate times the systolic pressure, the effect of the invention in lowering both pulse rate and systolic pressure leads to a significant reduction in heart load.

The pre-systolic blood pressure, i.e. the pressure at the points 98, 98', 98" in Fig. 3 seems to reduce by about -5 mm Hg for a probate with normal blood pressure of 120/60. Extremely beneficial is the fact that with patients with blood pressure which is too high the reduction is far more pronounced, although the reduction in the heart rate for such patients tends to be less than for normal patients.

It is also noted that the cardioresonance electrostimulation of the invention not only results in a lower systolic pressure but also a steeper pressure increase in the systole, which can also be seen from curve 90 in phase C of Fig. 3.

Generally speaking it can be said that DPTI increases by some +10 to 15 % depending on probates resulting from the hump in the blood pressure increase in diastole, reduced heart pulse rate and corrected by the difference from reduced pre-systolic blood pressure, assuming probates with normal blood pressure. TTI decreases by some 4 to 5 %, resulting from lower pre-systolic blood pressure corrected by the steeper pressure increase in systole (as shown at 7 in Fig. 3).

The benefit of this is that the DPTI / TTI ratio consequently increases by some 15 to 20 % depending on probates for those having normal blood pressure. Thus, the typical heart load reduction is some 10 to 25 % or more depending on the probates and their physical condition, which results from lower heart pulse rate and reduced systolic blood pressure and lower presystolic pressure. Furthermore, myocardial contractivity is improved, coronary blood circulation increased and ischemia reduced.

Turning now to Fig. 2a there can be seen a further sensor 90 which is connected by a lead 92 to a sensor signal processor 94 which is in turn connected, for the purpose of illustration, via a lead 96 to the electrocardiograph 28. The sensor 90 is a heart signal sensor of a non-electrical kind. It can for example be an acoustic sensor which detects the heart signal by the different acoustic noises generated during the operation of the heart. The acoustic signals are converted by a transducer into electrical signals and are processed in the device 94 to generate a signal corresponding to the electrocardiogram 32 shown in the electrocardiograph 28.

Various different types of non-electrical sensors 90 are known and all can be used for the purposes of the present invention. For example the non-electrical sensor can be selected from the group comprising a non-invasive, aortic pressure measurement device, an invasive aortic pressure measurement device and a noise detection device adapted to detect the closing of the heart valves. The benefit of using a non-electrical sensor is that the sensor is not disturbed by the electrical noise resulting from the electrical stimulation of the muscles. In fact, when such a sensor is used, the electrocardiograph 28 is actually redundant and therefore the lead 96 could lead, as is shown in dotted lines by the reference numeral 98 directly to the signal processor 36. Generally speaking the processor 36, which can be a PC, will be connected to a screen 100 with a keyboard 102 and the operator or physician 46 can then observe the heart trace on the screen (if desired) and can input parameters for the operation of the processor into the computer 36 via the keyboard. In addition reference numeral 104 signifies an internet connection which enables the physician or operator 46 to download new or updated operating programs for his electrotherapy apparatus which are made available as so-called firmware by the manufacturer of the electrotherapy apparatus.

A brief description will now be given as to how the processor 36 basically operates to provide electrical stimulation signals.

The processor either receives signals from the electrocardiograph 28 or from the non-electrical sensor 90 (or from both) and is programmed to recognize the R-R peaks of the electrocardiogram, these being the largest signal peaks and being the easiest to recognize. The processor first makes a determination for each successive pair of signal peaks of a value corresponding to the time between the successive pairs of signal peaks and thus to the persons heart rate. For example, if the patient has a regular heart beat of 60 beats per minute, then the time between successive pairs of R-R peaks is one second or 1000 milliseconds. Generally speaking a person's heart rate is not entirely regular and with many patients in need of treatment for heart problems it is definitely irregular. This means that the distance between successive pairs of signal peaks fluctuate and may vary significantly from the 1000 milliseconds of the example given above.

Once the R-R value is known for a particular heart cycle the processor is also able to calculate, using the known Bazett relationship, the number of milliseconds till the expected end of the T-wave for the next heart beat. Rather than calculating this value using the Bazett relationship the processor can also be programmed to look up the corresponding value in a suitable look up table or other statistical database.

Should the operator or physician decide that the stimulation will not be carried out precisely at the end of the T-wave, but at a slightly earlier or later time then he can input the required offset value (as a percentage) into the keyboard and it will be adopted by the system.

Instead of using a value of the preceding time between signal peaks as a value for the R-R path it is also possible to use an average value formed from a plurality of past values. In this way it is possible for the processor to be programmed to include in the plurality of past values those values which lie within a range less than the preceding measured value plus a predefined positive deviation and more than a value corresponding to the preceding measured value less a predefined deviation. This means that only reasonable values are taking into account in forming the average value and thus increases the reliability of the system.

Turning now to Figs. 5A and 5B an explanation will be given of a preferred form of the electrical stimulation applied to a person or patient during each heart cycle.

Fig. 5A shows the first train of pulses 44 comprising the individual biphasic pulses 44', 44", 44"', 44"" and 44""'.providing the initial stimulation. Each pulse of the train of pulses providing the initial electrical stimulation is a biphasic pulse having the same general form as shown in Fig. 2E. The frequency, i.e. the pulse repetition frequency of the pulses 44 providing the initial electrical stimulation, is selected in this example to be 150 Hz. This means that each pulse has a duration of 1000 ms divided by 150 = 6.66 ms. The width of one biphasic pulse, i.e. of one positive half wave and one negative half wave immediately following the positive half wave is 1 ms. Thus, if the time is started at the rising flank of the first biphasic pulse shown at the left-hand side of the diagram of Fig. 5A, then the value of 0 ms can be written beside it, as shown on the timescale beneath the start op the train of pulses 44 in Fig. 5A. Thus, the first biphasic pulse has terminated after 1 ms and the next starts at 6.666 ms so that there is an interval of 5.666 ms between the end of the first pulse 44' in Fig. 5A and the beginning of the next pulse 44" in Fig. 5A. The times at which the subsequent pulses 44", 44"', 44"" and 44""' start and finish are all entered on the timescale of Fig. 5A.

As can be seen from Fig. 5B the train of pulses 44 comprising the five individual pulses 44', 44", 44"', 44"", 44""' providing the initial electrical stimulation and generating the muscle contraction is followed by further stimulating electrical pulses 100, 100' etc. with a longer interval 102 between the individual pulses so that these are triggered at intervals 102 of 25 milliseconds corresponding to a pulse repetition frequency of 40 Hz. These further stimulating electrical pulses are intended to extend the muscle contraction up to a time shortly before the next R peak.

Thus, the main purpose is to control and extend the end of contraction of the muscles subjected to electrical stimulation thus improving hemodynamics by some 20 % while reducing electric loading of the human body.

The way this is achieved will be explained further with reference to Figs. 6 and 7.

Fig. 6 shows a diagram similar to the graph of Fig. 3 but also illustrating the muscle contraction achieved. Again, Fig. 6 is divided into three sections A, B and C, with section A representing the case of no electrical stimulation, section B representing the start of the electrical stimulation and section C the situation with continued electrical stimulation.

First a delay (Delay 1) to fit into the window (-5 % to +45 % of R-R measured from end of T-wave) is calculated based on the last measured R-R path length, or on an average value for the preceding R-R path length. A first short train of impulses (Train 1= 44 in Figs. 5A and 5B) is being delivered just sufficient to trigger a first short muscle contraction (Contraction 1). After a pause (defined by technical limits = 1 or more than one impulse) a new set of multiple very short trains (Train 2, 3....n) is delivered. In this example the "trains" 2, 3, ...n are further electrical stimulating pulses in the form of single biphasic pulses as shown by the further electrical stimulation pulses 100 in Fig. 5B. Each "train" 2, 3, ...n is just sufficient to maintain the muscle contraction, which is being perceived as one single muscle contraction. Based on experience, which results from measurements, the length of the first muscle contraction (Contraction 1) is calculated as having approximately three times the duration of train 1 and each subsequent very short train (Train 2, 3...n) of impulses triggers an additional muscle contraction wave, which is being superimposed on "Contraction 1". Again the additional increment of the muscle contraction in time can be calculated, because it is known that the descending slopes of "Contractions 2, 3... n" are creating a extension of the "Contraction 1" (= parallel shifting of "Contraction 1" on the time axis). By this method the total muscle contraction time resulting from "Trains 1 plus 2, 3,...n" can be calculated by the microprocessor to have the last extension of "Train n" and "Contraction n" to end within a window of 85 % - 95 % of the last (or average of more than one last) R-R1 (the last one available at the time of calculation which takes place after the detection of R-R1.

Thus, using the typical example of Figs. 5A and 5B: Train 1: Frequency 150 Hz, Duration = 3 % of R-R (allowing 5 impulses of 6.666 ms at impulse width of 1ms length and at e. g. 60 bpm heart rate, giving a duration = 30 ms), amplitude of e.g. 25 V, pause = 3 impulses of train 1 = 20 ms. This train 1 will trigger a muscle contraction of typically 90 ms "Contraction 1". Train 2 consequently will start after the elapsed time "Duration" of train 1, plus pause = 50 ms after the beginning of train 1 and consist of only one impulse at same amplitude, Train 3 is identical to train 2 and follows with a delay equal to the train repeat interval, i.e. every 25 ms a single impulse until n. In this example the trains 2, 3...n are always identical single impulses every 25 ms, which could be considered as a single second train of impulses from train 2 - n with a frequency of 40 Hz.

However this is only an example. The first train of pulses 44 could have less impulses than in above example, the minimum (one or more than one) impulses to trigger a first muscle contraction, the shape of the amplitudes within the train could have any form, the pause could have a duration corresponding to more or fewer impulses or could have a fixed time in ms, as long as a following train 2 would be able to maintain the Contraction 1 and add an increment of muscle contraction time. Moreover, the trains 2, 3, ... n could have a wider or more narrow time interval than the 24 milliseconds associated with the above 25 ms pulse repeat time (with a pulse width of one millisecond), again to maintain the incremental contraction 2 and add another increment of muscle contraction time. The Trains 2, 3...n could have one or more impulses, with any shape of amplitude within each train and furthermore the trains 2, 3,... n do not have to be identical.

The benefit of this technique is to minimize the electrical input (load) in to the human body and with this to avoid muscle fatigue and reduce adaptation of muscles to such a long stimulation, while achieving a muscle contraction time ending in a calculated window of 85 % - 95 % of R-R and giving at the same time an additional approximately 20 % improvement in hemodynamics. The same muscle contraction time could be achieved by simply extending train 1 to last as long as required. In above example the train 1 would have to be repeated and would have to deliver its last impulse at the moment of impulse n. Since the electric input is the integral of the amplitude for the impulse width times the number of impulses during the train it becomes clear that, in above example, the electric input for the invention with the multiple trains is only slightly above 20 % (actually the difference of electric input of train 1 to the average of the trains 2, 3..n) to achieve the same muscle contraction time.

With regard to a faster end of the descending slope of the muscle contraction it is favorable to use the shortest possible train 1 resulting in the "master" contraction 1, which is then maintained by the following short impulses. In above example, the contraction 1 lasts about 3 times the duration of train 1, with a descending slope of about 50 ms. This means that the muscle contraction n ends about 50 ms after the train n.
The shorter this descending train, the faster the muscle contraction increment can end.

It is now necessary to differentiate between two specific cases. In Fig 6 the muscle contraction time is calculated by the microprocessor to end in the time window calculated with R-R1 to be within 85 % -95 % of R-R1 after R1. The picture also shows the sequencing of the stimulation output signal from channel 1 to the electrode overlying a first muscle or muscle group 1, from channel 2 to an electrode overlying a second muscle or muscle group etc. Providing the next R peak, i.e. R2 occurs after the end of the predicted window, i.e. after 95 % of R-R1 after R1, all is well. However, R2 could occur before the end of the predicted window and therefore the muscle contraction could extend into the systolic phase of R2R3 which is undesirable. This situation is illustrated in Fig. 7.

In Fig 7 the muscle contraction time is extended (compared to Fig. 6) and is terminated with the last train n after the sensor has detected the following peak R2. The sensor has triggered trigger signal T2. At the first rising vertical slope of the trigger signal T2 the microprocessor of the device will end the continuation of adding additional trains (i.e. train n+1 is no longer transmitted). The muscle stimulation time consequently ends after the conclusion of the muscle contraction increment n added by train n has concluded the muscle contraction with the final descending slope.

It has to be noted that there is a technical time difference, called "Gap" in Fig. 7, which has to be observed. It represents the transmission delay time from the time the sensor detects the following R-wave (R2 in Fig. 7) to the time the first vertically rising slope of the trigger signal is processed by the microprocessor. A typical transmission delay is around 20 ms. Fig. 7 shows an example, where the train repeat interval, i.e. the time between train n and train n+1 is 25 ms. In this example, the vertical slope of the trigger signal T3 is being sent just shortly after the beginning of train n. The transmission delay of 20 ms is just smaller than the gap (time difference between the rising vertical slope of trigger T3 and the following train n+1) and in this example the microprocessor can just prevent train n+1. For cases in which the selected train repeat interval is smaller a smaller gap will result. Train n+1 can no longer be prevented by the microprocessor if the gap becomes smaller than the trigger signal transmission delay. For such cases, the microprocessor will prevent train n+2, meaning that the muscle contraction time will be extended by the train repeat interval.

Two cases need to be distinguished:
a) The case when an ECG signal is used for the determination of the R peaks.
   In this case, an electronic gate would have to be used to close the trigger signal input to the microprocessor exactly with the first impulse of each train and to reopen it immediately after the end of the last impulse width of each train. This is illustrated in Fig. 8 for a train x in Fig 12 in order to eliminate the possibility of self-induced triggering by the electrical impulse delivered. In the above example with a 25 ms train repeat interval and a single impulse per train with an impulse width of 1 ms and an assumed gate opening speed (processing time) of less than 0.5 ms, the ECG trigger window would be open for more than 23.5 ms or more than 94 % of the stimulation time in-between Train 2, 3 ... n. This is predictable but involves the risk that an R peak may be missed if it takes place at a time when the ECG trigger window is closed.
b) The case when a non-electrical sensor is used for the determination of the R peaks.

An additional, non-electrical sensor (e.g. triggering on the rising slope of a non-invasive aortic pressure measurement increase, or triggering on the noise detection of the closing of the heart valve etc.) would be used for applications for which an intermittently closed ECG trigger gate is unacceptable. By non-electrical sensor is meant a sensor which does not detect voltages or currents related to the operation of the heart but, for example, relies on pressure or noise measurements to sense the heart rhythm. The term non-electrical sensor does not exclude sensors which use electrical or electronic techniques to detect pressure or noise signals. A non-electrical sensor would not detect the stimulation impulse and consequently only trigger on the following P-wave or QRS complex, depending on whatever non-electrical sensing system is being used.

This sensor would not necessarily replace the ECG sensing in this invention, because the ECG display is being used to adjust the delay to the wanted time relative to the ECG (window of 5 % before and 45 % of R-R after the end of T-wave).

Turning now to Fig. 9 there is shown a lady who has been provided with a variety of electrodes 40', 40", 40"', 40"" and corresponding passive electrodes 42. The electrodes shown as circles with a white interior are associated with a group A of four channels. The circular electrodes with single hatching are associated with a group B of four channels and the four electrodes with cross-hatching are associated with group C of four channels. The passive electrodes 42 associated with each group of channels are shown as squares. They are electrically equivalent and are unhatched for channel group A, single hatched for channel group B and cross-hatched for channel group C.

The electrodes 40' and 40" of group A are provided over the left and right lateralis groups of muscles. The electrodes 40"' and 40"" of the group A are provided over the left and right glutea muscles. The passive electrodes 42 associated with group A are placed over the infra inguinalis muscles.

The electrodes 40' and 40" of group B are provided over the left and right femoralis medialis muscles whereas the electrodes 40"' and 40"" associated with group B are provided over the left and right sulcus glutealis muscles. The passive electrodes associated with channel group B are provided over the left and right supragenus muscles.

The electrodes 40' and 40" associated with channel group C are provided over the left and right medialio muscles whereas the electrodes 40"' and 40"" are provided over the left and right lateralis muscles of the calf. The passive electrodes 42 associated with channel group C are provided over the doralis pedis muscles on the left and right feet of the lady.

Thus, in this embodiment the circular non-hatched electrodes 40' to 40"" and the associated passive electrodes 42 are associated with the regio adominis/glutea muscles. The single hatched electrodes 40' to 40"' and the associated single hatched passive electrodes 42 are associated with the regio glutea femoralis muscles.

The double-hatched electrodes 40' to 40"" and the cross-hatched passive electrodes 42 are associated with the regio cruralis muscles.

There are several main ways of operating the electrotherapy apparatus with a patient provided with the electrodes as shown.

Before explaining how the electrotherapy apparatus is used in connection with Fig. 9 it is helpful to consider Fig. 10. This shows how, for the channel group A comprising channels 1 to 4, which are associated with the non-hatched electrodes 40' to 40"" of Fig. 9 the initial stimulating pulses 44 are applied from channel 1 to electrode 40', from channel 2 to electrode 40", from channel 3 to electrode 40"' and from channel 4 to electrode 40"", in each case at a time just after the end of the T-wave. Moreover it shows how, for the channel group B, comprising the channels 5, 6, 7 and 8, the initial stimulating signals transmitted by those channels are transmitted later than the initial stimulating signals transmitted by the corresponding channels of channel group B by an amount labeled offset. These signals are applied in the scheme of Fig. 9 to the single hatched electrodes 40' to 40"". Not shown in Fig. 10 is the channel group C comprising channels 9 to 12 which is associated with the cross-hatched electrodes 40' to 40"" and where the electrical stimulating signals are provided with an offset which is twice the value of the offset of the signals of channel B (the value twice is chosen arbitrarily and although preferred in this case is not to be understood to be restrictive). As before, the initial electrical stimulation is increased by further stimulating pulses with the aim of terminating the muscle contraction in each heart cycle just before the next R peak. Because the in initial stimulating signals start later in channels B and C than in channel A the total length of muscle contraction of the muscles associated with channels B and C will also be shorter than the muscle contraction associated with channel A, and indeed generally by the amount of the respective offset.

When the electrotherapy apparatus is operated in this mode then the effect will be to increase the pumping of blood from the heart to the periphery. This will lead to improved peripheral arterial perfusion and expediently also to an increase of venous return.

Another possibility exists of exploiting the electrodes in the arrangement shown in Fig. 9. In this case the electrodes are connected differently to the electrotherapy apparatus. More specifically, the non-hatched electrodes 40' to 40"" are connected to channels 9 to 12 of channel group C. The single hatched electrodes 40' to 40"" are connected to channels 5 to 8 of channel group B and the cross-hatched electrodes 40' to 40"" are connected to the channels 1 to 4 of channel group A. In each case only one channel is connected to any one electrode (as in the previous example).

With the electrodes connected in this way, and operating with the same offsets as shown in Fig. 10, i.e. with the stimulation signals being applied to the channels of channel group A at the end of the T-wave (or shortly thereafter) with the stimulation signals of channel group B being applied to respective electrodes at a later time with a suitable offset value, and with the stimulation signals of channel group C being applied to the associated electrodes with a larger offset time. The effect is to direct blood flow from the periphery back to the heart.

A similar effect can be achieved with only two channel groups A and B, by placing the electrodes of the channel group A either in the region cruralis or region glutea-femoralis and the electrodes of channel group B in the area of the region glutea-femoralis or region abdominis / glutea. essential is that the electrodes of the different groups are close in body areas which have a significant difference in their distance from the heart. To direct the effect from the periphery to the heart, the electrodes of the channel group stimulating later (due to the offset) are placed closer to the heart than the ones from the channel group stimulating earlier.

Alternatively, it is possible operate with no offset between the channel groups A and B, or A and B and C, if three groups of channels are provided.

The apparatus used to trigger the electrical stimulation pulses can be the apparatus described in the simultaneously filed application entitled "Electrotherapy Apparatus" published as WO-A-2005/044374.

Having described how the electrotherapy apparatus designed by the present applicants is contrived the specific realisation of the present invention will now be described. Thus the present teaching relates to electrotherapy apparatus for applying electrical stimulation to a muscle or group of muscles of a human or other mammal, wherein said electrical stimulation comprises trains of electrical pulses, said electrical stimulation having parameters comprising a pulse repetition frequency, a duration of each pulse or group of pulses and a time offset of the start of the train of pulses relative to a predicted end of a T-wave of an electrocardiogram derived from said human or mammal, said offset lying in a range from 5 % of the R-R path length of the preceding heart cycle, or of an average value of the R-R path lengths of a plurality of preceding heart cycles, or of a representative R-R path length, before the predicted end of said T-wave up to 45 % of the R-R path length of the preceding heart cycle, or of an average value of the R-R path lengths of a plurality of preceding heart cycles, or of a representative R-R path length, after the predicted end of the T-wave.

The present electrotherapy apparatus is adapted to vary said offset in accordance with a predetermined pattern, or randomly, within pre-specified limits in the course of a treatment extending over many heart cycles, typically over more than 15 minutes.

The amplitude of the stimulating pulses may also be varied. The variation can take place frequently, possibly for each new heart cycle or periodically, say every few minutes or randomly by adapting the microprocessor to select different amplitudes within a predetermined range and at predetermined or random time intervals using a random number generator. If the variation takes place frequently or less frequently then it can be effected by programming a suitable pattern of variation into the microprocessor. This can be done by the operator or a plurality of different patterns can be stored in the microprocessor and used in turn. The variation in amplitude can amount to a variation in peak voltage of said electrical stimulating pulses in a range from +1 or -1 V to + 10 or -10 V from a nominal value selected in the range from typically 10 to 50 V.

The pulse repetition frequency can lie in a range from 20 to 1000 Hz, preferably in a range from 30 to 250 Hz. The operator can for example command the microprocessor to vary the pulse repetition frequency during each heart cycle from one heart cycle to the next, or can command the microprocessor to vary the pulse repetition frequency after a predetermined or randomly selected number of cycles. The pulse repetition frequency or frequencies used during each heart cycle can also be varied randomly or in accordance with a predetermined pattern. Again the patterns can be stored in the microprocessor or the microprocessor can be provided with a program for generating random patterns within pre-specified ranges which are technically meaningful for the treatment concerned and within the technical capability of the electrotherapy apparatus, e.g. within its range of possible adjustments.

So far as the parameter "duration" is concerned this can be the duration of a single pulse, or the duration of a group of pulses, for example of the train 44, or the duration of two or more different groups of pulses such as the train 44 and the group of pulses 100. The total duration of one or more groups of pulses typically lies in a range from 30 ms to 600 ms and can be varied in this range. Again the variations in the parameter "duration can be selected randomly in a specific range, by suitable programming of the microprocessor or can be generated in accordance with predetermined patterns stored in the microprocessor.

The interval between successive stimulation pulses can also be varied within one heart cycle or from one heart cycle to another or can be periodically or randomly varied over a plurality of heart cycles. Again this variation can take place in accordance with predetermined patterns stored in the microprocessor or randomly within a certain range. In one heart cycle the individual pulses can have a duration varying from 0.1 ms to 50 ms and can be varied in this range or in a smaller range.

The offset lies within said range from 5 % of the R-R path length of the preceding heart cycle, or of an average value of the R-R path lengths of a plurality of preceding heart cycles, or of a representative R-R path length, before the predicted end of said T-wave up to 45 % of the R-R path length of the preceding heart cycle, or of an average value of the R-R path lengths of a plurality of preceding heart cycles, or of a representative R-R path length, after the predicted end of the T-wave, and can be varied in this range from one heart cycle to the next. Alternatively it can be varied only after a plurality of heart cycles, the number of which is selected randomly within a certain range.

It is considered particularly beneficial when a plurality of said parameters are varied simultaneously or at different times which can again be selected in accordance with patterns stored in the microprocessor or randomly within a certain range. Algorithms for selecting numbers randomly within a any desired range are well known in games of chance in connection with so-called payout tables. Since the random variation of parameters ultimately amounts to selecting numbers in a predetermined range, for example amplitudes within a range of volts or pulse repetition frequencies with a range of frequencies, the parallel to a random number generator operating within a specified range in a game of chance is apparent and it will be appreciated that the same type of algorithms can be stored in the microprocessor and operated automatically once this mode of operation is selected and the ranges have been defined by the operator or manufacturer.

## Claims

1. Electrotherapy apparatus for applying electrical stimulation to a muscle or group of muscles of a human or other mammal, wherein said electrical stimulation comprises trains of electrical pulses, said electrical stimulation having parameters comprising a pulse repetition frequency, a duration .of each pulse or group of pulses and a time offset of the start of the train of pulses relative to a predicted end of a T-wave of an electrocardiogram derived from said human or mammal, said offset lying in a range from 5 % of the R-R path length of the preceding heart cycle, or of an average value of the R-R path lengths of a plurality of preceding heart cycles, or of a representative R-R path length, before the predicted end of said T-wave up to 45 % of the R-R path length of the preceding heart cycle, or of an average value of the R-R path lengths of a plurality of preceding heart cycles, or of a representative R-R path length, after the predicted end of the T-wave, wherein
the electrotherapy apparatus is adapted to vary said offset in accordance with a predetermined pattern, or randomly, within pre-specified limits in the course of a treatment extending over several heart cycles, typically over more than 15 minutes, wherein the electrotherapy apparatus comprises a microprocessor adapted to carry out the variation either from one heart cycle to the next or after a plurality of heart cycles the number of which is selected randomly.

2. Electrotherapy apparatus in accordance with claim 1,
**characterised in that**
said pulse repetition frequency lies in a range from 20 to 1000 Hz, preferably in a range from 30 to 250Hz and **in that** said electrotherapy apparatus is adapted to additionally vary said pulse repetition frequency.

3. Electrotherapy apparatus in accordance with any one of the preceding claims,
**characterised in that**
said pulse duration lies in the range from 0. 1 ms to 600 ms and said electrotherapy apparatus is adapted to additionally vary said pulse duration in this range.

4. Electrotherapy apparatus in accordance with any one of the preceding claims,
**characterised in that**
an interval between successive pulses can lie in a range from 0.1 ms to 50 ms and said electrotherapy apparatus is adapted to additionally vary said interval in this range.

## Patentansprüche

1. Elektrotherapievorrichtung zum Anwenden einer elektrischen Stimulation auf einen Muskel oder eine Gruppe von Muskeln eines Menschen oder eines anderen Säugetiers, wobei die elektrische Stimulation Folgen von elektrischen Impulsen umfasst, wobei die elektrische Stimulation Parameter aufweist, die eine Impulswiederholungsfrequenz, eine Dauer jedes Impulses oder jeder Gruppe von Impulsen und einen Zeit-Offset des Beginns der Folge von Impulsen relativ zu einem vorhergesagten Ende einer T-Welle eines von dem Menschen oder Säugetier abgeleiteten Elektrokardiogramms umfassen, wobei der Offset in einem Bereich von 5 % der R-R-Pfadlänge des vorhergehenden Herzzyklus oder eines Mittelwerts der R-R-Pfadlängen mehrerer vorhergehender Herzzyklen oder einer repräsentativen R-R-Pfadlänge vor dem vorhergesagten Ende der T-Welle bis zu 45 % der R-R-Pfadlänge des vorhergehenden Herzzyklus oder eines Mittelwerts der R-R-Pfadlängen mehrerer vorhergehender Herzzyklen oder einer repräsentativen R-R-Pfadlänge hinter dem vorhergesagten Ende der T-Welle liegt, wobei
die Elektrotherapievorrichtung geeignet ist, um den Offset gemäß einem vorbestimmten Muster, oder zufällig, innerhalb vorab spezifizierter Grenzen im Laufe einer Behandlung, die sich über mehrere Herzzyklen erstreckt, typischerweise über mehr als 15 Minuten, zu verändern,
wobei die Elektrotherapievorrichtung einen Mikroprozessor umfasst, der geeignet ist, um die Veränderung entweder von einem Herzzyklus zum Nächsten oder nach einer Mehrzahl von Herzzyklen, deren Anzahl zufällig ausgewählt wird, auszuführen.

2. Elektrotherapievorrichtung nach Anspruch 1,
**dadurch gekennzeichnet, dass**
die Impulswiederholungsfrequenz in einem Bereich von 20 bis 1000 Hz, vorzugsweise in einem Bereich von 30 bis 250 Hz, liegt, und dass die Elektrotherapievorrichtung geeignet ist, um ferner die Impulswiederholungsfrequenz zu verändern.

3. Elektrotherapievorrichtung nach einem der vorhergehenden Ansprüche,
**dadurch gekennzeichnet, dass**
die Impulsdauer in dem Bereich von 0,1 ms bis 600 ms liegt, und dass die Elektrotherapievorrichtung geeignet ist, um ferner die Impulsdauer in diesem Bereich zu verändern.

4. Elektrotherapievorrichtung nach einem der vorhergehenden Ansprüche,
**dadurch gekennzeichnet, dass**
ein Intervall zwischen aufeinanderfolgenden Impulsen in einem Bereich von 0,1 ms bis 50 ms liegen kann und dass die Elektrotherapievorrichtung geeignet ist, um ferner das Intervall in diesem Bereich zu verändern.

## Revendications

1. Appareil d'électrothérapie pour appliquer une stimulation électrique à un muscle ou un groupe de muscles d'un être humain ou d'un autre mammifère, dans lequel ladite stimulation électrique comprend des trains d'impulsions électriques, ladite stimulation électrique ayant des paramètres comprenant : une fréquence de répétition d'impulsions, une durée de chaque impulsion ou groupe d'impulsions, et un offset temporel du départ du train d'impulsions par rapport à une fin prévisible d'une onde T d'un électrocardiogramme dérivé depuis ledit être humain ou mammifère, ledit offset étant situé dans une plage de 5 % de la longueur du trajet R-R du cycle cardiaque précédent, ou d'une valeur moyenne des longueurs de trajets R-R d'une pluralité de cycles cardiaques précédents, ou d'une longueur de trajet R-R représentative, avant la fin prévisible de ladite onde T, jusqu'à 45 % de la longueur de trajet R-R du cycle cardiaque précédent, ou d'une valeur moyenne des longueurs de trajets R-R d'une pluralité de cycles cardiaques précédents, ou d'une longueur de trajet R-R représentative, après la fin prévisible de l'onde T, dans lequel
l'appareil d'électrothérapie est adapté à faire varier ledit offset en accord avec un motif prédéterminé, ou au hasard, dans des limites prédéterminées au cours d'un traitement s'étendant sur plusieurs cycles cardiaques, typiquement sur plus de 15 minutes,
dans lequel l'appareil d'électrothérapie comprend un microprocesseur adapté à effectuer la variation soit d'un cycle cardiaque au suivant soit après une pluralité de cycles cardiaques, dont le nombre est choisi au hasard.

2. Appareil d'électrothérapie selon la revendication 1, **caractérisé en ce que** ladite fréquence de répétition d'impulsions se trouve dans une plage de 20 à 1000 Hz, de préférence dans une plage de 30 à 250 Hz, et **en ce que** ledit appareil d'électrothérapie et adapté à faire additionnellement varier ladite fréquence de répétition d'impulsions.

3. Appareil d'électrothérapie selon l'une quelconque des revendications précédentes,
**caractérisé en ce que** ladite durée d'impulsion est située dans la plage de 0,1 ms à 600 ms, et ledit appareil d'électrothérapie et adaptée à faire additionnellement varier ladite durée d'impulsion dans cette plage.

4. Appareil d'électrothérapie selon l'une quelconque des revendications précédentes,
**caractérisé en ce qu'**un intervalle entre impulsions successives peut être situé dans une plage de 0,1 ms à 50 ms et ledit appareil d'électrothérapie est adapté à faire additionnellement varier ledit intervalle dans cette plage.
